# EUROPÄISCHE PATENTSCHRIFT

(11) **EP 4 547 116 B1**
(45) Veröffentlichungstag und Bekanntmachung des Hinweises auf die Patenterteilung: **08.04.2026**
(21) Anmeldenummer: 23735242.2
(22) Anmeldetag: 20.06.2023
(51) Int. Cl.: A61B 10/00, A61B 5/15, B01L 3/00

(54) **VERFAHREN ZUR HERSTELLUNG EINES DRUCKAUSGLEICHS SOWIE ZUGEHÖRIGES SET**
METHOD FOR PRODUCING A PRESSURE EQUALISATION AND ASSOCIATED SET
PROCÉDÉ DE PRODUCTION D'UNE ÉGALISATION DE PRESSION ET ENSEMBLE ASSOCIÉ

(30) Priorität: 28.06.2022 DE 102022116057
(43) Veröffentlichungstag der Anmeldung: 07.05.2025
(73) Patentinhaber: Sarstedt AG & Co. KG, 51588 Nümbrecht (DE)
(72) Erfinder: WEINSTOCK, Mark, 57612 Helmenzen (DE); KASPEREIT, Knut, 50670 Köln (DE)
(74) Vertreter: Bauer PSU PartG mbB
(86) Internationale Anmeldenummer: PCT/EP2023/066581
(87) Internationale Veröffentlichungsnummer: WO 2024/002780

(56) Entgegenhaltungen:
- WO-A1-2022/006258
- US-A1- 2014 054 330
- US-B2- 6 874 656
- US-B2- 7 070 065

## Beschreibung

Die vorliegende Anmeldung betrifft ein Set gemäß dem Oberbegriff von Anspruch 1. Des Weiteren betrifft die Anmeldung ein Verfahren zur Herstellung eines Druckausgleichs gemäß Anspruch 13.

Das Set umfasst mindestens ein Behältnis sowie mindestens einen Deckel zum Verschließen des Behältnisses. Das Behältnis des Sets umfasst wiederum einen Innenraum, in dem eine Körperflüssigkeit verwahrt werden kann. Bei der zu verwahrenden Körperflüssigkeit kann es sich insbesondere um Blut, Urin, Speichel oder eine andere von einem Patienten erhobene Körperflüssigkeit handeln. In aller Regel ist vorgesehen, dass die Körperflüssigkeit nach Abgabe in das Behältnis gegeben und in diesem bis zu einer Analyse verwahrt werden soll. Der Deckel des Sets sorgt dabei für eine sichere Verwahrung. Das Set ist mindestens zwischen einer Dichtstellung und einer Belüftungsstellung überführbar. In der Dichtstellung des Sets bilden das Behältnis und der Deckel gemeinsam einen luftdichten Dichtsitz aus. Mit anderen Worten steht der Deckel derart mit dem Behältnis in Kontakt, dass ein Austritt von Luft aus dem Behältnis verhindert ist. Insbesondere ist hierdurch auch ein Austritt der Körperflüssigkeit aus dem Innenraum des Behältnisses verhindert. Das Set wird typischerweise nach der Entnahme der Körperflüssigkeit in die Dichtstellung überführt, um einen sicheren Transport bzw. eine sichere Verwahrung der Körperflüssigkeit zu ermöglichen.

Weiterhin weist das Set eine Entnahmeeinheit auf. Die Entnahmeeinheit ermöglicht eine Entnahme der Körperflüssigkeit aus dem Innenraum des Behältnisses, auch wenn sich dieses in der Dichtstellung befindet, der Deckel das Behältnis also luftdicht verschließt. Mittels der Entnahmeeinheit wird typischerweise eine zu analysierende Menge der Körperflüssigkeit aus dem Behältnis entnommen, wobei die Entnahmeeinheit eine besonders einfache Entnahme der Körperflüssigkeit aus dem Behältnis ermöglicht, da ein Entfernen des Deckels von dem Behältnis hierfür nicht nötig ist. Die Entnahme der Körperflüssigkeit kann dabei mittels eines Entnahmemittels erfolgen, beispielsweise einer Spritze mit oder ohne eine aufgesteckten Kanüle, das durch die Entnahmeeinheit hindurch in den Innenraum des Behältnisses eingeführt werden kann, um von dort aus die Körperflüssigkeit abzusaugen.

Bei Vorliegen eines Unterdrucks innerhalb des Innenraums des Behältnisses infolge der Entnahme der Körperflüssigkeit durch die Entnahmeeinheit aus dem Behältnis ist bzw. sind der Deckel und/oder das Behältnis durch den Druckunterschied zwischen dem Innenraum des Behältnisses und der Umgebung so verformbar, dass das Set von der Dichtstellung in die Belüftungsstellung überführbar ist, wobei in der Belüftungsstellung des Sets Luft aus der Umgebung in den Innenraum des Behältnisses einströmt und den Innenraum des Behältnisses belüftet.

### Stand der Technik

Ein Set der eingangs beschrieben Art ist im Stand der Technik bereits bekannt. Hierzu wird insbesondere auf die "V-Monovette Urin" der Anmelderin hingewiesen. Bekannte Sets zur Verwahrung einer Körperflüssigkeit mit einer Entnahmeeinheit zur Entnahme der Körperflüssigkeit zeigen allerdings das Problem, dass infolge einer Entnahme der Körperflüssigkeit aus dem Behältnis, beispielsweise mittels Absaugens mittels einer Spritze, ein Druckunterschied zwischen dem Innenraum des Behältnisses und einer Umgebung des Sets aufgebaut wird. Der Druck der Umgebung entspricht dabei in aller Regel dem atmosphärischen Druck. Bei Erreichen eines bestimmten Unterdrucks ist ein weiteres Absaugen der Körperflüssigkeit aus dem Behältnis verhindert, da ein Nachströmen von Luft aus der Umgebung in den Innenraum des Behältnisses aufgrund des luftdichten Dichtsitzes zwischen dem Deckel und dem Behältnis verhindert ist. Auch ein nach Nachströmen von Luft über die Entnahmeeinheit ist verhindert. Um ein weiteres Absaugen der Körperflüssigkeit zu ermöglichen, muss das Behältnis zunächst erst belüftet werden. Das Set muss mithin in die Belüftungsstellung überführt werden. Hierzu muss der Deckel des Sets von dem Behältnis entfernt werden, um Luft in den Innenraum des Behältnisses einströmen lassen zu können und somit den Druckunterschied auszugleichen. Da der Deckel typischerweise auf das Behältnis aufgeschraubt ist, hat sich ein solches Vorgehen als mühselig und zeitaufwendig erwiesen. Zusätzlich kann es bei dem erneuten Öffnen des Behältnisses auch zu einem versehentlichen Verschütten der Körperflüssigkeit kommen. Die Entnahme der Körperflüssigkeit kann mithin lediglich portionsweise erfolgen und wird durch das händische Belüften des Behältnisses unterbrochen.

US6874656 B2 offenbart den Oberbegriff von Anspruch 1.

### Aufgabe

Der vorliegenden Anmeldung liegt mithin die Aufgabe zugrunde, ein Set hervorzubringen, das eine einfache Entnahme der Körperflüssigkeit aus dem Behältnis bei Vorliegen des Sets in der Dichtstellung ermöglicht.

### Lösung

Die zugrunde liegende Aufgabe wird erfindungsgemäß mittels eines Sets mit den Merkmalen des Anspruchs 1 gelöst. Vorteilhafte Ausgestaltungen ergeben sich aus den zugehörigen Unteransprüchen.

Das Set ist dadurch gekennzeichnet, dass der Deckel mindestens ein Belüftungselement aufweist, das bei Vorliegen des Sets in der Belüftungsstellung infolge der Verformung des Deckels und/oder des Behältnisses aufgrund eines Kontakts mit dem Behältnis und/ oder dem Deckel mindestens einen Spalt in dem Dichtsitz zwischen dem Deckel und dem Behältnis erzeugt, durch welchen Luft aus der Umgebung in den Innenraum des Behältnisses strömen kann.

Das erfindungsgemäß Set hat viele Vorteile. Insbesondere ermöglicht das Set eine Entnahme der Körperflüssigkeit aus dem Innenraum des Behältnisses über die Entnahmeeinheit, bei dem ein Entfernen des Deckels von dem Behältnis zur Ausbildung eines Druckausgleichs nicht nötig ist. Vielmehr hat das Set eine selbstbelüftende Funktion. Mit anderen Worten ist ein händisches Zutun nicht nötig, um den Innenraum des Behältnisses zu belüften. Infolge einer Entnahme der Körperflüssigkeit, beispielsweise zu Zwecken der weiteren Verarbeitung und/oder Untersuchung der Körperflüssigkeit, verformt bzw. verformen sich der Deckel und/oder das Behältnis, da die Entnahme aufgrund des luftdichten Dichtsitzes zwischen Deckel und Behältnis zu einem Druckunterschied zwischen dem Innenraum des Behältnisses und einer Umgebung führt. Insbesondere erfolgt infolge der Entnahme der Körperflüssigkeit aus dem Behältnis eine Druckverringerung des innerhalb des Behältnisses vorliegenden Drucks, sodass der Innenraum einem Unterdruck ausgesetzt ist. Dieser führt zu der eingangs genannten Verformung des Deckels und/oder des Behältnisses.

So ist vorstellbar, dass der Deckel eine im Wesentlichen plane Deckelbasis und einen Deckelrand aufweist, wobei sich die Deckelbasis infolge des Unterdrucks innerhalb des Innenraums des Behältnisses derart verformt, dass die Deckelbasis eine Wölbung aufweist. Infolge der Verformung des Deckels und/oder des Behältnisses wird das Set in die Belüftungsstellung überführt. In der Belüftungsstellung des Sets ist ein Einströmen von Luft aus der Umgebung des Sets ermöglicht, sodass der Innenraum des Behältnisses belüftet wird. Sobald der Druckunterschied zwischen dem Innenraum und der Umgebung aufgehoben wurde, ist das Set wieder selbstständig in die Dichtstellung überführbar, da die Verformung des Deckels und/oder des Behältnisses lediglich infolge des Druckunterschieds erfolgt ist, der jedoch nach der Belüftung aufgehoben wurde. Ein Einströmen von Luft in den Innenraum des Behältnisses ist mithin lediglich dann ermöglicht, wenn sich das Set in der Belüftungsstellung befindet. In der Dichtstellung hingegen ist diese gerade nicht gewünscht. Vorteilhafter Weise kann mittels des Sets eine beliebige Menge der Körperflüssigkeit aus dem Behältnis entnommen werden, ohne händisch für eine Belüftung des Innenraums zu sorgen.

Das Set kann neben der Dichtstellung und der Belüftungsstellung weitere Stellungen aufweisen, insbesondere diejenige Stellung, in der der Deckel von dem Behältnis entfernt ist. Diese Stellung des Sets kann dazu genutzt werden, die Körperflüssigkeit in das Behältnis einzufüllen. Nach erfolgter Einfüllung kann das Set in die Dichtstellung überführt werden, indem der Deckel mit dem Behältnis in Kontakt gebracht wird, beispielsweise auf dieses aufgeschraubt wird.

Das Belüftungselement wird vorzugsweise infolge der Verformung des Deckels und/oder des Behältnisses gegen den Deckel und/oder das Behältnis gedrückt. Beispielsweise ist vorstellbar, dass der Deckel aufgrund des Unterdrucks derart verformt wird, dass sich die Deckelbasis des Deckels wölbt, wobei der Deckelrand aufgrund der Verformung der Deckelbasis ebenfalls zumindest teilweise verformt wird und ein sich verformender Bereich des Deckelrands den Spalt in dem Dichtsitz erzeugt. Bei ansteigendem Unterdruck innerhalb des Behältnisses vergrößert sich der entstehende Spalt. Als Folge kann Luft aus der Umgebung in den Innenraum des Behältnisses einströmen und das Behältnis belüften. Infolge der einströmenden Luft wird der Druckunterschied zwischen dem Innenraum und der Umgebung wieder aufgehoben. Vorzugsweise wird das Set infolge der Belüftung und einer Verformung des Deckels und/oder des Behältnisses in eine Ausgangsstellung zurück in die Dichtstellung überführt, in der der Dichtsitz zwischen Deckel und dem Behältnis vorliegt.

Dabei ist gemäß einer vorzugsweisen Ausgestaltung der Erfindung vorgesehen, dass das Belüftungselement in Form einer Nocke ausgebildet ist. In Versuchen hat sich herausgestellt, dass ein Belüftungselement in Form einer Nocke besonders gut dazu geeignet ist, einen Spalt in dem Dichtsitz zwischen Deckel und Behältnis zu erzeugen. Die Nocke kann dabei vorzugsweise tropfenförmig ausgebildet sein und derart an dem Deckel des Sets angeordnet sein, dass eine breitere Seite der tropfenförmigen Nocke bei der Verformung des Deckels mit dem Behältnis in Kontakt kommt und somit den Spalt ausbildet.

Gemäß einer vorteilhaften Ausgestaltung der Erfindung ist dabei vorgesehen, dass die Nocke an einer dem Behältnis bei Vorliegen des Sets in der Dichtstellung zugewandten Unterseite des Deckels angeordnet ist. In der Dichtstellung des Sets verschließt der Deckel das Behältnis und eine Unterseite des Deckels ist vorzugsweise dem Innenraum des Deckels zugewandt. Bei einer Verformung des Deckels und/oder des Behältnisses kann sich ein Abstand zwischen der Nocke und einer Wandung des Behältnisses derart reduzieren, dass die Nocke mit der Wandung des Behältnisses in Kontakt kommt und bei einer weiteren Reduzierung des Abstands den Spalt in dem Dichtsitz ausbildet. Die Anordnung der Nocke an der Unterseite des Deckels hat sich dabei als besonders vorteilhaft für den Fall erwiesen, dass sich der Deckel infolge des Druckunterschieds verformt. Dabei sollte die Nocke derart an der Unterseite des Deckels angeordnet sein, dass die Nocke in einem verschlossenen Zustand des Behältnisses, d.h., wenn der Deckel auf dem Behältnis angeordnet ist, und sich das Set in der Dichtstellung befindet, keinen Kontakt zu dem Behältnis hat. Erst bei Vorliegen eines Unterdrucks innerhalb des Innenraums des Behältnisses sollte die Nocke mit dem Behältnis, insbesondere dessen Wandung, in Kontakt kommen und das Set in die Belüftungsstellung überführen. Der in dem Behältnis vorherrschende Unterdruck, ab dem eine Verformung des Deckels stattfindet, kann dabei durch die Wahl eines Materials des Deckels und/oder des Behältnisses vorbestimmt werden. Auf diese Weise kann sichergestellt werden, dass sich der Deckel nicht bereits bei Vorliegen sehr geringer Unterdrücke verformt. Nach Einströmen der Luft aus der Umgebung in den Innenraum des Behältnisses erfolgt vorzugsweise eine Rückverformung des Deckels in eine Ausgangsstellung, in der die Nocke nicht mit dem Behältnis in Kontakt kommt. Das Set liegt mithin wieder in der Dichtstellung vor.

Eine vorzugsweise Ausgestaltung sieht vor, dass die Entnahmeeinheit rohrförmig ausgebildet ist, wobei die Entnahmeeinheit an einem ersten Ende offen ausgebildet und an einem zweiten Ende mit einer bei und nach Durchstechen selbstverschließenden Membran versehen ist. Vorzugsweise ist dabei vorgesehen, dass die Körperflüssigkeit mittels eines Entnahmemittels, beispielsweise einer Spitze, die auch eine Kanüle aufweisen kann, aus dem Behältnis entnehmbar ist. Eine rohrförmige Ausgestaltung der Entnahmeeinheit ist dabei besonders von Vorteil, da sich die Spritze, insbesondere bei Verwendung mit einer Kanüle, besonders einfach in das Behältnis einführen lässt, da die Entnahmeeinheit dabei als Führung wirkt. Dabei erstreckt sich die Entnahmeeinheit vorzugsweise bis unmittelbar vor einen Boden des Behältnisses, sodass sichergestellt ist, dass die Körperflüssigkeit auch dann aus dem Behältnis entnehmbar ist, wenn ein Flüssigkeitsspiegel sehr niedrig ist. In jedem Fall muss jedoch sichergestellt werden, dass die Entnahme derart erfolgt, dass die in dem Behältnis zu verwahrende Körperflüssigkeit während und nach der Entnahme nicht ungewollt aus dem Behältnis austritt. Hierzu hat sich die selbstverschließende Membran als besonders vorteilhaft erwiesen, da ein händisches Zutun eines Verwenders des Sets nicht nötig ist. Die Membran verschließt den Innenraum dabei sowohl bei als auch nach Durchstechen der Membran luftdicht gegenüber der Umgebung, sodass während der Entnahme der Körperflüssigkeit keine Luft aus der Umgebung in den Innenraum des Behältnisses oder in umgekehrter Richtung aus dem Innenraum in die Umgebung gelangen kann. Zugleich ermöglicht die selbstverschließende Membran die Verwahrung einer Flüssigkeitsmenge, die einen Flüssigkeitspegel, der dem zweiten Ende entspricht, übersteigt. Mit anderen Worten kann das Behältnis mit einer beliebigen Menge der Körperflüssigkeit gefüllt werden, ohne dass die Körperflüssigkeit über die Entnahmeeinheit aus dem Behältnis austreten kann.

Eine vorteilhafte Ausgestaltung der Erfindung sieht vor, dass die Membran eine vorbestimmte Einstichstelle aufweist, wobei die Membran im Bereich der Einstichstelle vorzugsweise geschlitzt ist. Mittels Ausbildung einer vorbestimmten Einstichstelle ist sichergestellt, dass ein Verwender des Sets die Membran der Entnahmeeinheit nicht beliebig einstechen oder durchstechen muss. Vielmehr gibt die vorbestimmte Einstichstelle vorteilhafter Weise vor, wo das Entnahmemittel durchzuführen ist. Für den Fall, dass die Membran im Bereich der Einstichstelle geschlitzt ist, ist eine Einführung des Entnahmemittels zusätzlich vereinfacht, da dieses dann lediglich durch die geschlitzte Einstichstelle eingeführt werden muss. Dabei ist vorzugsweise vorgesehen, dass die geschlitzte Einstichstelle dennoch während und nach der Entnahme mittels des Entnahmemittels einen luft- und flüssigkeitsdichten Abschluss zwischen dem Innenraum des Behältnisses und der Umgebung sicherstellt.

Eine besonders vorteilhafte Weiterentwicklung des erfindungsgemäßen Verfahrens sieht vor, dass das Behältnis becherförmig ausgebildet ist. Typischerweise sind Behältnisse zur Verwahrung von Körperflüssigkeiten aufgrund der einfachen Handhabung becherförmig ausgebildet. Dabei kann vorzugsweise vorgesehen sein, dass sich ein horizontaler Querschnitt des Behältnisses in Längsrichtung des Behältnisses vergrößert. Ebenso kann vorgesehen sein, dass der Querschnitt in Längsrichtung konstant bleibt.

Vorzugsweise kann der Deckel einen kreisförmigen Querschnitt aufweisen. Entsprechend kann das Behältnis becherförmig ausgebildet sein und ebenfalls einen kreisförmigen Querschnitt aufweisen, sodass der Deckel und das Behältnis aufeinander abgestimmt sind.

Eine weitere Ausgestaltung der Erfindung sieht vor, dass der Deckel mittels eines Befestigungsmittels an dem Behältnis haltbar ist, wobei das Befestigungsmittel vorzugsweise ein Schraubgewinde ist. Mittels eines Schraubgewindes ist der Deckel besonders einfach, sicher und schnell an dem Behältnis zu befestigen. Das Schraubgewinde kann auf einfache Weise sicherstellen, dass die in dem Behältnis untergebrachte Körperflüssigkeit nicht auslaufen kann. Mithilfe der Entnahmeeinheit ist dabei dennoch eine Entnahme aus dem Behältnis ermöglicht, ohne den Deckel von dem Behältnis abschrauben zu müssen.

Ferner ist es besonders vorteilhaft, wenn ein Durchmesser des Deckels größer ist als ein Durchmesser des Behältnisses, sodass der Deckel das Behältnis in der Dichtstellung des Sets zumindest teilweise umgreift. Für den Fall, dass der Deckel eine Deckelbasis und einen Deckelrand aufweist, ist eine derartige Ausgestaltung besonders von Vorteil, da die Deckelbasis das Behältnis, insbesondere in der Dichtstellung, so verschließt und der Deckelrand das Behältnis umgreift. Dabei kann vorzugsweise vorgesehen sein, dass das Befestigungsmittel des Deckels an einer Innenfläche des Deckelrands angeordnet ist. Gleichzeitig kann das Behältnis in einem korrespondierenden Bereich ebenfalls ein Befestigungsmittel aufweisen. Beispielswese ist vorstellbar, dass der Deckel ein Schraubgewinde aufweist, das mit einem korrespondierenden Gewinde, welches an einem oberen Rand des Behältnisses angeordnet ist, mit dem Behältnis verschraubbar ist.

Eine vorteilhafte Ausgestaltung der Erfindung sieht vor, dass ein Öffnungsquerschnitt der Entnahmeeinheit in einer Ebene mit einer Deckeloberfläche, insbesondere einer Unterseite des Deckels, liegt. Mit anderen Worten ist die Deckeloberfläche plan ausgebildet. Auf diese Weise ist das Set besonders kompakt ausgebildet und lässt sich einfach stapeln.

Eine weitere Ausgestaltung der Erfindung sieht vor, dass der Deckel mindestens einen Luftströmungskanal aufweist, durch welchen Luft bei Vorliegen des Sets in der Belüftungsstellung vor oder nach Durchströmen des Spaltes aus der Umgebung in den Innenraum des Behältnisses strömen kann. Bei einem bestimmungsgemäßen Gebrauch des Sets kann vorgesehen sein, dass der Deckel in der Dichtstellung auf das Behältnis aufgeschraubt ist, wobei der Deckel nicht unmittelbar auf einer Stirnfläche des Behältnisses aufliegt. Vorzugsweise steht der Deckel lediglich über den Deckelrand mit dem Behältnis in Kontakt. Eine Stirnfläche des Behältnisses steht dabei nicht in Kontakt mit einer Unterseite des Deckels. Für den Fall, dass der Deckel jedoch derart fest mit dem Behältnis verschraubt wurde, dass ein Kontakt zwischen der Stirnfläche des Behältnisses und der Deckelbasis des Deckels hergestellt wurde, besteht das Risiko, dass das Behältnis nicht belüftet werden kann, da ein Formschluss zwischen der Deckelbasis und der Wandung des Behältnisses vorliegt. In einem solchen Fall ist der Luftströmungskanal besonders von Vorteil, da dieser es ermöglicht, Luft auch dann in den Innenraum des Behältnisses einströmen zu lassen, wenn das Set fälschlicherweise auf die vorbeschriebene Weise zusammengesetzt wurde. Der Luftströmungskanal ist dabei vorzugsweise einem Belüftungselement zugeordnet, sodass Luft bei Vorliegen des Sets in der Belüftungsstellung zunächst über den Luftströmungskanal und anschließend über den durch das Belüftungselement ausgebildeten Spalt in den Innenraum des Behältnisses strömen kann.

Gemäß einer vorteilhaften Ausgestaltung des erfindungsgemäßen Sets ist ferner vorgesehen, dass der Luftströmungskanal in einem Kontaktbereich des Deckels und des Behältnisses in der Dichtstellung des Sets, insbesondere in einer umlaufenden, ringförmigen Stirnfläche des Behältnisses, angeordnet ist. Diese Anordnung ermöglicht eine Belüftung des Innenraums des Behältnisses für den Fall, dass der Deckel derart auf dem Behältnis angeordnet ist, dass ein Einströmen aufgrund eines Kontakts zwischen dem Deckel und dem Behältnis in dem Kontaktbereich verhindert ist.

Die eingangs genannte Aufgabe wird zudem gemäß dem Verfahren mit den Merkmalen des Anspruchs 13 gelöst. Eine vorteilhafte Ausgestaltung ergibt sich aus dem zugehörigen Unteranspruch.

Das Verfahren eignet sich zur Herstellung eines Druckausgleichs zwischen einem Innenraum eines Behältnisses eines Sets. Das Verfahren umfasst dabei die folgenden Verfahrensschritte: Zunächst befinden sich eine Körperflüssigkeit und Luft in dem Innenraum des Behältnisses. Dabei ist das Set in der Dichtstellung, in der ein Austreten der Körperflüssigkeit und der Luft aufgrund eines Dichtsitzes zwischen dem Deckel und dem Behältnis des Sets verhindert ist.

In einem zweiten Verfahrensschritt wird die Körperflüssigkeit aus dem Innenraum des Behältnisses entnommen. Hierzu wird die Entnahmeeinheit des Sets genutzt. Beispielsweise kann die Entnahme der Körperflüssigkeit mittels eines Entnahmemittels in Form einer Spritze aus dem Innenraum des Behältnisses abgesogen werden. Infolge dieser Entnahme wird ein Druck, der im Innenraum des Behältnisses vorliegt, verringert, da ein Nachströmen von Luft als Ausgleich für die Entnahme der Körperflüssigkeit aufgrund des Dichtsitzes zwischen dem Deckel und dem Behältnis verhindert ist. Als Ergebnis liegt in dem Innenraum des Behältnisses ein Unterdruck gegenüber der Umgebung vor. Als Folge des Unterdrucks wird bzw. werden der Deckel und/oder das Behältnis des Sets verformt.

In einem dritten Verfahrensschritt wird das Set infolge der Verformung von der Dichtstellung in die Belüftungsstellung überführt, in der Luft aus der Umgebung in den Innenraum des Behältnisses einströmt und den Innenraum des Behältnisses belüftet. Als Folge der Belüftung wird das Set zurück in die Dichtstellung überführt.

Die in Bezug auf das erfindungsgemäße Set genannten Vorteile werden dabei analog von dem vorbeschriebenen Verfahren erzielt.

Eine vorzugsweise Ausgestaltung der Erfindung sieht vor, dass das Set automatisch zwischen der Dichtstellung und der Belüftungsstellung überführt wird. Mit anderen Worten erfolgt die Überführung des Sets zwischen den Stellungen ohne händisches Zutun. Ein Verwender des Sets kann die Körperflüssigkeit mithin besonders einfach aus dem Behältnis entnehmen, ohne hierbei ergänzende Handlungen zur Belüftung des Sets vornehmen zu müssen, da sich das Set selbst belüftet. Insbesondere ist es nicht nötig, den Deckel des Sets während der Entnahme zur Belüftung des Innenraums von dem Behältnis zu entfernen. Die Entnahme der Körperflüssigkeit aus dem Innenraum des Behältnisses erfolgt vielmehr ohne Unterbrechungen, da sich das Set selbst belüftet, sobald der in dem Innenraum des Behältnisses vorherrschende Druck einen Grenzwert unterschreitet. Der Grenzwert kann dabei vorzugsweise durch die Wahl eines Materials des Deckels und/oder des Behältnisses vorbestimmt sein.

### Ausführungsbeispiele

Das erfindungsgemäße Set sowie das erfindungsgemäße Verfahren werden nachstehend anhand eines Ausführungsbeispiels, das in den Figuren dargestellt ist, näher erläutert. Es zeigt:
- Fig. 1:: Eine dreidimensionale Ansicht eines erfindungsgemäßen Sets.
- Fig. 2:: Eine Unterseite eines Deckels des Sets aus Figur 1.
- Fig. 3a: Eine Detailansicht der Unterseite des Deckels aus Figur 2, wobei das Set in einer Dichtstellung vorliegt.
- Fig. 3b: Eine Detailansicht der Unterseite des Deckels aus Figur 2, wobei das Set in einer Belüftungsstellung vorliegt.
- Fig. 4: Einen vertikalen Schnitt durch das Set aus Figur 1.
- Fig. 5: Einen weiteren vertikalen Schnitt durch das Set aus Figur 1.
- Fig. 6a: Eine Detailansicht des Schnitts aus Figur 5.
- Fig. 6b: Eine weitere Detailansicht des Schnitts aus Figur 5, wobei ein Luftführungskanal des Sets geöffnet ist.
- Fig. 7: Einen vertikalen Schnitt durch eine Entnahmeeinheit des Sets aus Figur 1.

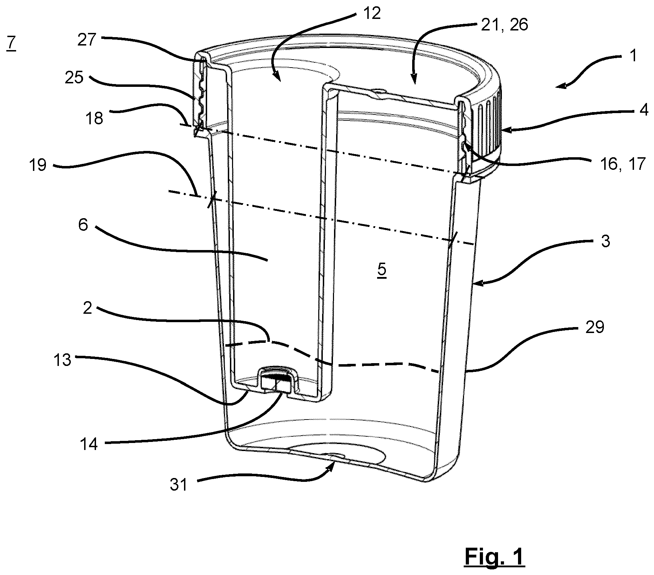

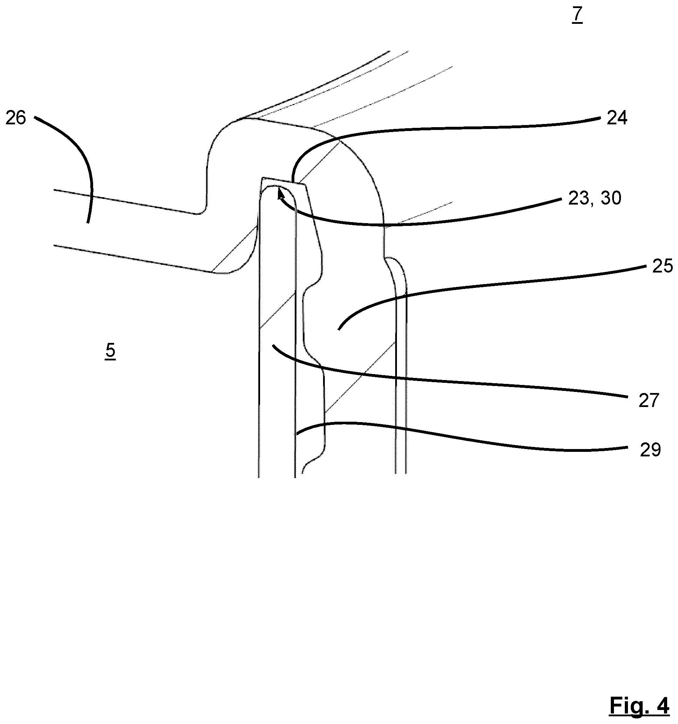

Ein Ausführungsbeispiel, das in den **Figuren 1 bis 4** gezeigt ist, umfasst ein erfindungsgemäßes Set **1.** Das Set **1** dient zur Aufbewahrung einer Körperflüssigkeit **2,** beispielsweise Urin. Das Set **1** umfasst ein Behältnis **3** zur Verwahrung mindestens einer Körperflüssigkeit **2** und einen Deckel **4.** Das Behältnis **3** ist becherförmig ausgebildet und weist einen kreisförmigen, sich in Richtung einer Oberseite **32** des Behältnisses **3** vergrößernden Querschnitt auf. Der Deckel **4** ist dazu vorgesehen, das Behältnis **3** luftdicht zu verschließen. Hierzu ist der Deckel **4** ebenfalls in seinem Querschnitt kreisförmig ausgebildet, wobei ein Durchmesser **18** des Deckels **4** größer ist als ein Durchmesser **19** des Behältnisses **3,** sodass der Deckel **4** auf das Behältnis **3** aufgelegt werden kann. Weiterhin umfasst der Deckel **4** einen Deckelrand **25,** der sich senkrecht zu einer Deckelbasis **26** des Deckels **4** erstreckt. Auf diese Weise umgreift der Deckel **4** das Behältnis **3** zumindest teilweise, wenn er auf dem Behältnis **3** aufliegt.

Um einen luftdichten Dichtsitz zwischen Deckel **4** und Behältnis **3** ausbilden zu können, weisen der Deckel **4** und das Behältnis **3** jeweils ein Befestigungsmittel **16** auf, welches in Form eines Schraubgewindes **17** ausgebildet ist. Auf diese Weise lässt sich der Deckel **4** einfach und schnell auf das Behältnis **3** aufschrauben. Dabei ist das Schraubgewinde **17** des Behältnisses **3** an einem oberen umlaufenden Rand **27** an einer Außenseite des Behältnisses **3** angeordnet ist. Das Schraubgewinde **17** des Deckels **4** ist an einer Innenseite des Deckelrands **25** angeordnet. In einem aufgesetzten Zustand umgreift der Deckelrand **25** das Behältnis **3.** Das Behältnis **3** und der Deckel **4** lassen sich dann mittels einer Verschraubung des Deckels **4** auf dem Behältnis **3** in eine luftdichte Dichtstellung des Sets **1** überführen, in der ein Austritt und Einströmen von Luft und ein Austritt der Körperflüssigkeit **2** aus dem Innenraum **5** des Behältnisses **3** verhindert ist.

Das Set **1** ist zwischen der Dichtstellung und einer Belüftungsstellung überführbar. Darüber hinaus kann das Set **1** auch in einer Ausgangsstellung vorliegen, in der der Deckel **4** von dem Behältnis **3** getrennt ist. In der Dichtstellung ist der Deckel **4** auf das Behältnis **3** aufgeschraubt und ein Entweichen von Luft oder der Körperflüssigkeit **2** ist verhindert. Die Dichtstellung ist in der **Figur 1** gezeigt.

Weiterhin umfasst das Set **1** eine Entnahmeeinheit **6.** Die Entnahmeeinheit **6** ermöglicht eine Entnahme der Körperflüssigkeit **2** bei aufgeschraubtem Deckel **4.** Diese ist rohrförmig ausgebildet, wobei ein erstes Ende **12** der Entnahmeeinheit **6** offen ausgebildet ist, während das gegenüberliegende zweite Ende **13** der Entnahmeeinheit **6** mit einer Membran **14** versehen ist, die sich beim und nach Durchstechen selbstständig verschließt. Die Membran **14** verhindert ebenso wie der Dichtsitz einen Austritt und ein Einströmen von Luft aus oder in den Innenraum. Auch ist die Membran **14** für die Körperflüssigkeit **2** undurchlässig. Ein Öffnungsquerschnitt **20** der Entnahmeeinheit **6** liegt dabei in einer Ebene mit einer Deckeloberfläche **21,** nämlich der Unterseite **11** des Deckels **4.** Der Deckel **4** ist mithin trotz Vorhandensein der Entnahmeeinheit **6** eben ausgebildet. Ein Öffnungsquerschnitt **20** der Entnahmeeinheit **6** ist dezentral von einem Mittelpunkt **28** des Deckels **4** bzw. des Behältnisses **3** angeordnet, sodass eine geringe Menge der Körperflüssigkeit **2** unter Neigung des Behältnisses **3** ebenfalls mittels der Entnahmeeinheit **6** aus dem Behältnis **3** entnehmbar ist.

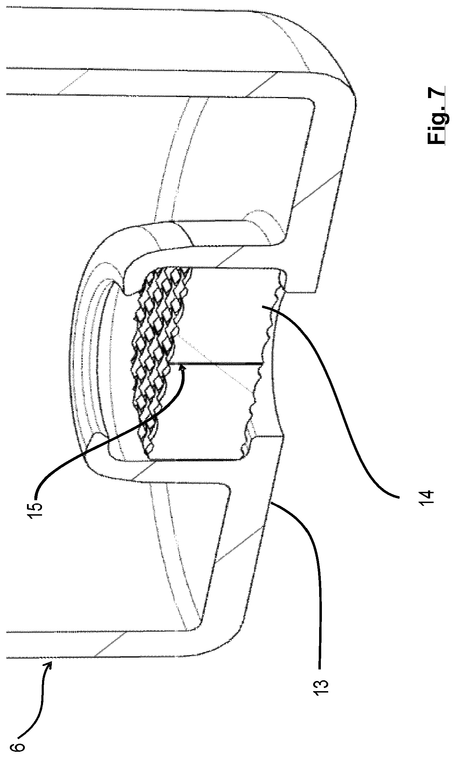

Um eine bestimmte Menge zur Analyse der Körperflüssigkeit **2** aus dem Behältnis **3** zu entnehmen, kann die Entnahmeeinheit **6** genutzt werden. Hierzu kann ein Entnahmemittel, beispielsweise eine Spritze mit oder ohne Kanüle, durch die Membran **14** hindurch in die Körperflüssigkeit **2** eingeführt werden. Beim Erreichen der am Ende der Entnahmeeinheit **6** angeordneten Membran **14** wird die Spritze durch eine vorgegebene Einstichstelle **15** hindurchgeführt, wie insbesondere aus der Figur 7 ersichtlich ist. Mittels eines auf das Entnahmemittel händisch ausgeübten Unterdrucks, insbesondere durch Aufziehen der Spritze, kann die Körperflüssigkeit **2** aus dem Behältnis **3** über die Entnahmeeinheit **6** herausgesogen werden. Das Entnahmemittel kann mithin bei aufgeschraubtem Deckel **4** besonders einfach durch die Entnahmeeinheit **6** hindurch in den Innenraum **5** eingeführt werden, wobei die selbstverschließende Membran **14** sicherstellt, dass die Körperflüssigkeit **2** auch beim Durchstechen der Membran **14** luftdicht von der Umgebung **7** abgetrennt ist. Gleichermaßen verschließt sich die Membran **14** nach dem Durchstechen selbstständig wieder. Die Dichtstellung wird mithin auch dann beibehalten, wenn die Spritze sich in dem Innenraum **5** befindet.

Mittels der Spritze kann die Körperflüssigkeit **2** aus dem Innenraum **5** des Behältnisses abgesaugt werden und so eine bestimmte Menge der Körperflüssigkeit **2** entnommen werden. Während des Absaugens der Körperflüssigkeit **2** verringert sich der in dem Innenraum **5** des Behältnisses **3** vorherrschende Druck aufgrund des luftdichten Verschlusses des Behältnisses **3** mittels des Deckels **4.** In dem Innenraum **5** des Behältnisses **3** bildet sich ein Unterdruck gegenüber der Umgebung **7** des Sets **1** aus. Die Verringerung des Drucks bewirkt eine steigend erschwerte Entnahme der Körperflüssigkeit **2** aus dem Behältnis **3,** da ein Nachströmen von Luft als Reaktion auf die entnommene Körperflüssigkeit **2** aufgrund der Dichtstellung des Sets **1** verhindert ist. Bei Vorherrschen eines bestimmten Unterdrucks innerhalb des Innenraums **5** ist eine weitere Entnahme der Körperflüssigkeit **2** mittels Absaugens schließlich nicht mehr möglich.

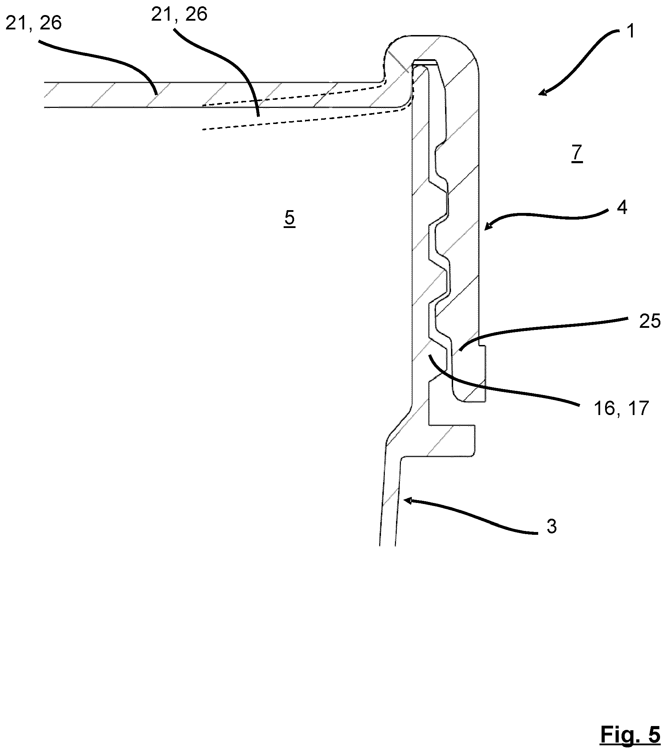

Um dies zu verhindern, wird bei Erreichen eines vorbestimmten Druckunterschieds zwischen dem in dem Innenraum **5** vorherrschenden Druck und dem der Umgebung **7,** welcher dem atmosphärischen Druck entspricht, der Deckel **4** des Sets 1 verformt. Insbesondere wölbt sich die Deckelbasis **26** in Richtung eines Bodens **31** des Behältnisses **3,** wie besonders gut in **Figur 5** ersichtlich ist. Der Druckunterschied, bei dem eine solche Verformung des Deckels **4** stattfindet, ist dabei mittels einer Wahl eines eingesetzten Materials für die Ausbildung des Deckels **4** und/oder des Behältnisses **3** anpassbar.

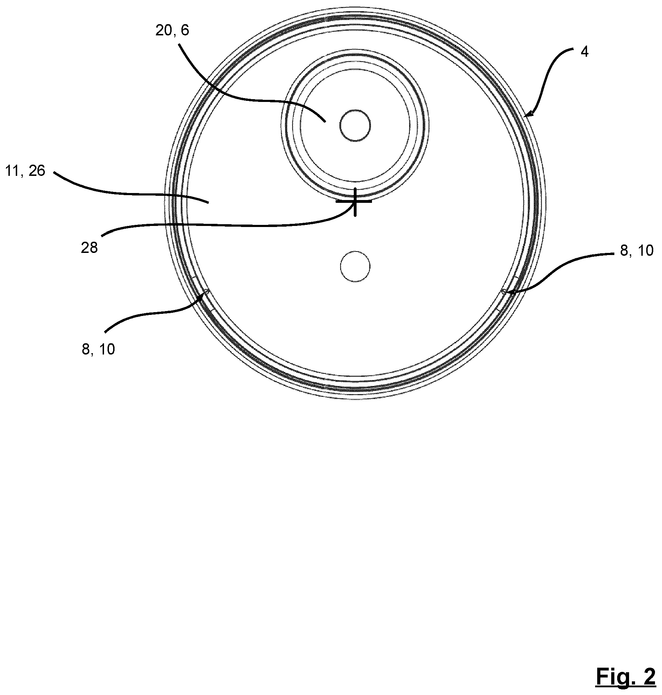

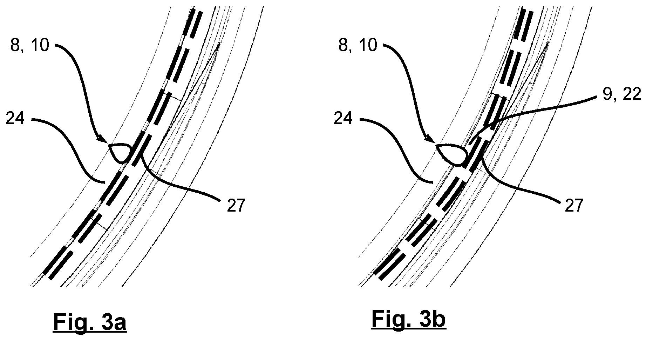

Der Deckel **4** des Sets **1** weist zwei Belüftungselemente **8** auf, die an einer Unterseite **11** des Deckels **4** in einem Randbereich des selbigen angeordnet sind. Die Belüftungselemente **8** sind jeweils in Form einer Nocke **10** ausgebildet, wie besonders gut aus den **Figuren 2** **und** **3** ersichtlich ist.

Infolge der Verformung des Deckels **4** kommen die Nocken **10** jeweils mit einer Wandung **29** des Behältnisses in Kontakt. Auf diese Weise wird ein Spalt **9** zwischen dem Deckel **4** und dem Behältnis **3** ausgebildet, wie in **Figur 3b****)** ersichtlich ist. Das Set **1** wird in diesem Zuge von der Dichtstellung in eine Belüftungsstellung überführt, in der ein Einströmen von Luft aus der Umgebung **7** in den Innenraum **5** des Behältnisses **3** ermöglicht ist. Als Folge wird der Innenraum **5** des Behältnisses **3** belüftet und der Unterdruck aufgehoben, sodass eine Entnahme der Körperflüssigkeit **2** mittels Absaugens dieser aus dem Innenraum **5** wieder ermöglicht wird. Während des kontinuierlichen Druckausgleichs infolge des Einströmens der Luft aus der Umgebung **7** verformt sich der Deckel **4** zurück in eine Ausgangsstellung und der Spalt **9** wird in einer Größe verringert. Bei einem vollständig erfolgten Druckausgleich mit der Umgebung **7** ist der Spalt 9 vollständig verschlossen und das Set **1** befindet sich wieder in der Dichtstellung, wie insbesondere in **Figur 3a****)** ersichtlich ist. Die Verformung des Deckels **4** und die damit einhergehende Ausbildung des Spalts **9** erfolgt automatisch, d.h. ohne händisches Zutun. Ein Verwender des Sets **1** kann die Körperflüssigkeit **2** somit besonders einfach aus dem Innenraum **5** des Behältnisses **3** absaugen, ohne hierbei durch Entfernen des Deckels **4** von dem Behältnis **3** für einen Druckausgleich zwischen dem Innenraum **5** des Behältnisses **3** und der Umgebung **7** sorgen zu müssen. Vielmehr erfolgt das Absaugen der Körperflüssigkeit **2** aus dem Innenraum **5** des Behältnisses **3,** ohne dass der Verwender des Sets **1** eine Veränderung bemerkt.

Dieser Vorgang, also die Überführung zwischen Dichtstellung und Belüftungsstellung des Sets **1,** wiederholt sich in Abhängigkeit der Menge der zu entnehmenden Körperflüssigkeit **2.** Die Menge der Körperflüssigkeit **2,** die entnommen wird, bevor das Set **1** in automatisch in die Belüftungsstellung überführt wird, ist dabei im Wesentlichen stets gleich. Allerdings nimmt ein Verwender die Überführung zwischen der Dichtstellung und der Belüftungsstellung nicht wahr, da er nicht eingreifen muss.

Vorgesehen ist, dass der Deckel **4** derart aufgeschraubt wird, dass zwischen einer Stirnfläche **30** des Behältnisses **3** und der Stirnfläche **24** des Deckels **4** ein umlaufender Spalt **9** verbleibt, sodass die Stirnflächen **24, 30** nicht miteinander in Kontakt kommen. Ein Kontakt soll lediglich im Bereich der beiden Schraubgewinde **17** vorliegen.

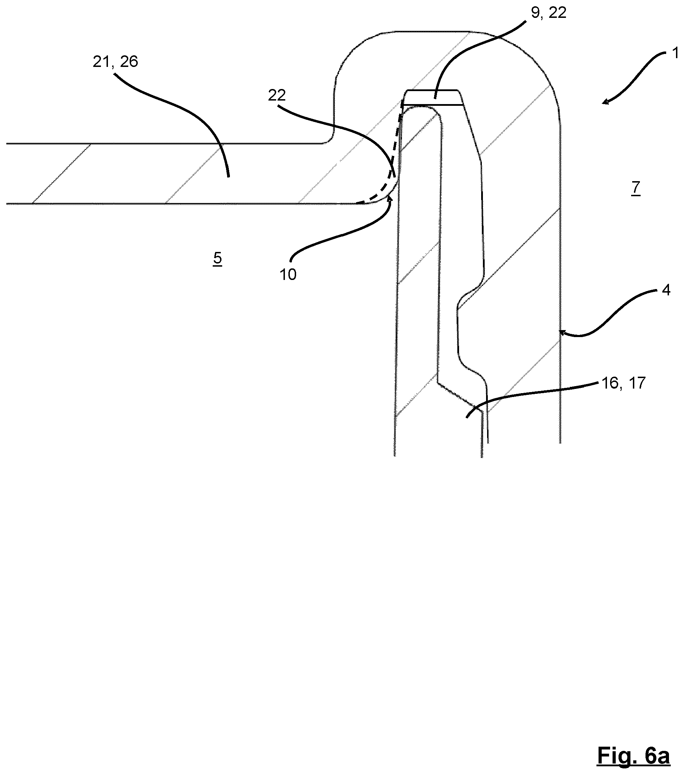

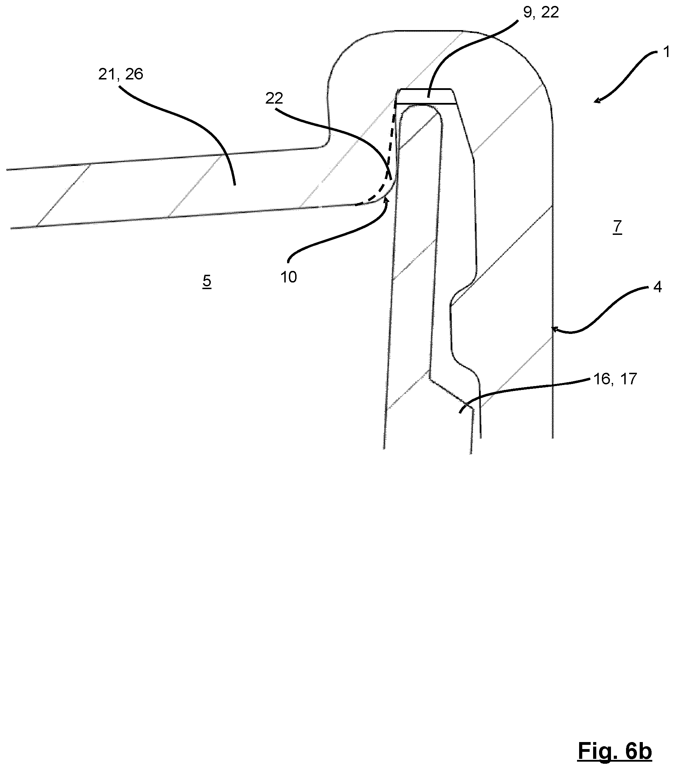

Allerdings hat es sich herausgestellt, dass der Deckel **4** derart falsch aufgeschraubt werden kann, dass die beiden Stirnflächen **24, 30** zumindest teilweise miteinander in Kontakt kommen. Eine solche Verschraubung ist insbesondere in den Figuren 6a) und 6b) ersichtlich. In einem derartigen Fall kann der Innenraum **5** des Behältnisses **3** auch bei Vorliegen des Sets **1** in der Belüftungsstellung nicht oder zumindest nicht ausreichend schnell belüftet werden, da ein Einströmen der Luft infolge des Kontakts der beiden Stirnflächen **24, 30** gehemmt oder verhindert ist. Für diesen Fall ermöglicht ein Luftströmungskanal **22,** der an der Stirnfläche **24** des Deckels **4** angeordnet ist, dass Luft vor Durchströmen des Spalts **9** aus der Umgebung **7** in den Innenraum **5** des Behältnisses **3** strömen kann. Hierzu ist der Luftströmungskanal **22** in einem Kontaktbereich **23** des Deckels **4** und des Behältnisses **3** in der Dichtstellung des Sets **1** angeordnet. Der Luftströmungskanal **22** ist dabei der jeweiligen Nocke **10** zugeordnet. Bei einem fehlerhaften Aufschrauben des Deckels **4** auf das Behältnis **3** kann das Set **1** so dennoch in die Belüftungsstellung überführt werden.

### Bezugszeichenliste

- 1: Set
- 2: Körperflüssigkeit
- 3: Behältnis
- 4: Deckel
- 5: Innenraum
- 6: Entnahmeeinheit
- 7: Umgebung
- 8: Belüftungselement
- 9: Spalt
- 10: Nocke
- 11: Unterseite des Deckels
- 12: Erstes Ende
- 13: Zweites Ende
- 14: Membran
- 15: Einstichstelle
- 16: Befestigungsmittel
- 17: Schraubgewinde
- 18: Durchmesser des Deckels
- 19: Durchmesser des Behältnisses
- 20: Öffnungsquerschnitt der Entnahmeeinheit
- 21: Deckeloberfläche
- 22: Luftströmungskanal
- 23: Kontaktbereich
- 24: Stirnfläche des Deckels
- 25: Deckelrand
- 26: Deckelbasis
- 27: Rand des Behältnisses
- 28: Mittelpunkt
- 29: Wandung
- 30: Stirnfläche der Wandung
- 31: Boden des Behältnisses
- 32: Oberseite des Behältnisses

## Patentansprüche

1. Set (1), umfassend
- mindestens ein Behältnis (3) zur Verwahrung mindestens einer Körperflüssigkeit (2) und
- mindestens einen Deckel (4) zum Verschließen des Behältnisses (3),
- wobei das Behältnis (3) mindestens einen Innenraum (5) zur Verwahrung der Körperflüssigkeit (2) aufweist,
- wobei das Set (1) mindestens zwischen einer Dichtstellung und einer Belüftungsstellung überführbar ist,
- wobei der Deckel (4) und das Behältnis (3) in der Dichtstellung des Sets (1) gemeinsam einen luftdichten Dichtsitz ausbilden,
- wobei der Deckel (4) eine Entnahmeeinheit (6) aufweist, mittels der die Körperflüssigkeit (2) auch bei Vorliegen des Sets (1) in der Dichtstellung aus dem Behältnis (3) entnehmbar ist,
- der Deckel (4) und/oder das Behältnis (3) bei Vorliegen eines Unterdrucks innerhalb des Innenraums (5) des Behältnisses (3) infolge der Entnahme der Körperflüssigkeit (2) durch die Entnahmeeinheit (6) aus dem Behältnis (3) durch den Druckunterschied zwischen dem Innenraum (5) des Behältnisses (3) und der Umgebung (7) so verformbar ist bzw. sind, dass das Set (1) von der Dichtstellung in die Belüftungsstellung überführbar ist, wobei in der Belüftungsstellung des Sets (1) Luft aus der Umgebung (7) in den Innenraum (5) des Behältnisses (3) einströmt und den Innenraum (5) des Behältnisses (3) belüftet,
**dadurch gekennzeichnet, dass**
der Deckel (4) mindestens ein Belüftungselement (8) aufweist, das bei Vorliegen des Sets (1) in der Belüftungsstellung infolge der Verformung des Deckels (4) und/oder des Behältnisses (3) aufgrund eines Kontakts mit dem Behältnis (3) und/oder dem Deckel (4) mindestens einen Spalt (9) in dem Dichtsitz zwischen dem Deckel (4) und dem Behältnis (3) erzeugt, durch welchen Luft aus der Umgebung (7) in den Innenraum (5) des Behältnisses (3) strömen kann.

2. Set (1) nach Anspruch 1, **dadurch gekennzeichnet, dass** das Belüftungselement (8) in Form einer Nocke (10) ausgebildet ist.

3. Set (1) nach Anspruch 2, **dadurch gekennzeichnet, dass** die Nocke (10) an einer dem Behältnis (3) bei Vorliegen des Sets (1) in der Dichtstellung zugewandten Unterseite (11) des Deckels (4) angeordnet ist.

4. Set (1) nach einem der vorhergehenden Ansprüche, **dadurch gekennzeichnet, dass** die Entnahmeeinheit (6) rohrförmig ausgebildet ist, wobei die Entnahmeeinheit (6) an einem ersten Ende (12) offen ausgebildet und an einem zweiten Ende (13) mit einer bei und nach Durchstechen selbstverschließenden Membran (14) versehen ist.

5. Set (1) nach Anspruch 4, **dadurch gekennzeichnet, dass** die Membran (14) eine vorbestimmte Einstichstelle (15) aufweist, wobei die Membran (14) im Bereich der Einstichstelle (15) vorzugsweise geschlitzt ist.

6. Set (1) nach einem der vorhergehenden Ansprüche, **dadurch gekennzeichnet, dass** das Behältnis (3) becherförmig ausgebildet ist.

7. Set (1) nach einem der vorhergehenden Ansprüche, **dadurch gekennzeichnet, dass** der Deckel (4) einen kreisförmigen Querschnitt aufweist.

8. Set (1) nach einem der vorhergehenden Ansprüche, **dadurch gekennzeichnet, dass** der Deckel (4) mittels eines Befestigungsmittels (16) an dem Behältnis (3) haltbar ist, wobei das Befestigungsmittel (16) vorzugsweise ein Schraubgewinde (17) ist.

9. Set (1) nach einem der vorhergehenden Ansprüche, **dadurch gekennzeichnet, dass** ein Durchmesser (18) des Deckels (4) größer ist als ein Durchmesser (19) des Behältnisses (3) (19), sodass der Deckel (4) das Behältnis (3) in der Dichtstellung des Sets (1) zumindest teilweise umgreift.

10. Set (1) nach einem der vorhergehenden Ansprüche, **dadurch gekennzeichnet, dass** ein Öffnungsquerschnitt (20) der Entnahmeeinheit (20) (6) in einer Ebene mit einer Deckeloberfläche (21), insbesondere einer Unterseite (11) des Deckels (4), liegt.

11. Set (1) nach einem der vorhergehenden Ansprüche, **dadurch gekennzeichnet, dass** der Deckel (4) mindestens einen Luftströmungskanal (22) aufweist, durch welchen Luft bei Vorliegen des Sets (1) in der Belüftungsstellung vor oder nach Durchströmen des Spaltes (9) aus der Umgebung (7) in den Innenraum (5) des Behältnisses (3) strömen kann.

12. Set (1) nach Anspruch 11, **dadurch gekennzeichnet, dass** der Luftströmungskanal (22) in einem Kontaktbereich (23) des Deckels (4) und des Behältnisses (3) in der Dichtstellung des Sets (1), insbesondere in einer umlaufenden, ringförmigen Stirnfläche (30) des Behältnisses (3), angeordnet ist.

13. Verfahren zur Herstellung eines Druckausgleichs zwischen einem Innenraum (5) eines Behältnisses (3) eines Sets (1) nach einem der Ansprüche 1 bis 12 und einer Umgebung (7), umfassend die folgenden Verfahrensschritte:
- Das Set (1) befindet sich in der Dichtstellung und in dem Innenraum (5) des Behältnisses (3) befinden sich eine Körperflüssigkeit (2) und Luft.
- Ein Druck im Innenraum (5) des Behältnisses (3) wird infolge einer Entnahme einer Körperflüssigkeit (2) durch die Entnahmeeinheit (6) hindurch aus dem Innenraum (5) verringert, sodass gegenüber einer Umgebung (7) des Sets (1) ein Unterdruck im Innenraum (5) des Behältnisses (3) entsteht und der Deckel (4) und/oder das Behältnis (3) des Sets (1) verformt wird bzw. werden.
- Infolge der Verformung wird das Set (1) von der Dichtstellung in die Belüftungsstellung überführt, in der Luft aus der Umgebung (7) in den Innenraum (5) des Behältnisses (3) einströmt und den Innenraum (5) des Behältnisses (3) belüftet, wodurch das Set (1) zurück in die Dichtstellung überführt wird.

14. Verfahren nach Anspruch 13, **dadurch gekennzeichnet, dass** das Set (1) automatisch zwischen der Dichtstellung und der Belüftungsstellung überführt wird.

## Claims

1. A set (1), comprising
- at least one container (3) for storing at least one bodily fluid (2), and
- at least one lid (4) for closing the container (3),
- wherein the container (3) has at least one interior (5) for storing the bodily fluid (2),
- wherein the set (1) can be transferred at least between a sealing position and a ventilating position,
- wherein the lid (4) and the container (3) together form an airtight sealing seat in the sealing position of the set (1),
- wherein the lid (4) has a removal unit (6), by means of which the bodily fluid (2) can be removed from the container (3) even with the set (1) in the sealing position,
- given a vacuum inside of the interior (5) of the container (3) as a result of removing the bodily fluid (2) from the container (3) via the removal unit (6), the pressure differential between the interior (5) of the container (3) and the environment (7) makes it possible to deform the lid (4) and/or the container (3), such that the set (1) can be transferred from the sealing position into the ventilating position, wherein, with the set (1) in the ventilating position, air flows into the interior (5) of the container (3) from the environment (7) and ventilates the interior (5) of the container (3),
**characterized in that**
a lid (4) has at least one ventilating element (8), which, when the set (1) is in the ventilating position, generates at least one gap (9) in the sealing seat between the lid (4) and the container (3) as a result of the deformation of the lid (4) and/or the container (3) due to contact with the container (3) and/or the lid (4), through which air can flow from the environment (7) into the interior (5) of the container (3).

2. The set (1) according to claim 1, **characterized in that** the ventilating element (8) is designed as a cam (10).

3. The set (1) according to claim 2, **characterized in that** the cam (10) is arranged on a lower side (11) of the lid (4) that faces the container (3) with the set (1) in the sealing position.

4. The set (1) according to one of the preceding claims, **characterized in that** the removal unit (6) is tubular in design, wherein the removal unit (6) is open at a first end (12), and provided with a membrane (14) at a second end (13) that self-seals while and after being pierced.

5. The set (1) according to claim 4, **characterized in that** the membranae (14) has a predetermined puncture site (15), wherein the membrane (14) is preferably slit in the area of the puncture site (15).

6. The set (1) according to one of the preceding claims, **characterized in that** the container (3) is cup-shaped.

7. The set (1) according to one of the preceding claims, **characterized in that** the lid (4) has a circular cross section.

8. The set (1) according to one of the preceding claims, **characterized in that** the lid (4) can be held on the container (3) by means of a fastener (16), wherein the fastener (16) is preferably a screw thread (17).

9. The set (1) according to one of the preceding claims, **characterized in that** a diameter (18) of the lid (4) is larger than a diameter (19) of the container (3) (19),so that the lid (4) at least partially envelops the container (3) in the sealing position of the set (1).

10. The set (1) according to one of the preceding claims, **characterized in that** an opening cross section (20) of the removal unit (20) (6) lies in a plane with a lid surface (21), in particular a lower side (11) of the lid (4).

11. The set (1) according to one of the preceding claims, **characterized in that** the lid (4) has at least one air flow channel (22), though which air can flow from the environment (7) into the interior (5) of the container (3) before or after flowing through the gap (9) with the set (1) in the ventilating position.

12. The set (1) according to claim 11, **characterized in that** the air flow channel (22) is arranged in a contact area (23) of the lid (4) and the container (3) in the sealing position of the set (1), in particular in a circumferential, ring-shaped end face (30) of the container (3).

13. A method for establishing a pressure equalization between an interior (5) of a container (3) of a set (1) according to one of claims 1 to 12 and an environment (7), comprising the following procedural steps:
- The set (1) is in the sealing position, and a bodily fluid (2) and air are in the interior (5) of the container (3).
- A pressure in the interior (5) of the container (3) is decreased by removing a bodily fluid (2) from the interior (5) through the removal unit (6), so that a vacuum in the interior (5) of the container (3) is created in relation to an environment (7) of the set (1), and the lid (4) and/or the container (3) of the set (1) is deformed.
- Due to the deformation, the set (1) is transferred from the sealing position into the ventilating position, in which air flows from the environment (7) into the interior (5) of the container (3), and ventilates the interior (5) of the container (3), as a result of which the set (1) is transferred back into the sealing position.

14. The method according to claim 13, **characterized in that** the set (1) is automatically transferred between the sealing position and the ventilating position.

## Revendications

1. Kit (1), comprenant
- au moins un contenant (3), destiné à conserver au moins un fluide corporel (2) et
- au moins un couvercle (4), destiné à fermer le contenant (3),
- le contenant (3) comportant au moins un espace intérieur (5), destiné à conserver le fluide corporel (2),
- le kit (1) pouvant être transféré au moins entre une position d'étanchéité et une position d'aération,
- dans la position d'étanchéité du kit (1), le couvercle (4) et le contenant (3) constituant conjointement un siège d'étanchéité étanche à l'air,
- le couvercle (4) comportant une unité de prélèvement (6), à l'aide de laquelle le fluide corporel (2) peut être prélevé hors du contenant (3), même si le kit (1) se trouve dans la position d'étanchéité,
- en présence d'une dépression à l'intérieur de l'espace intérieur (5) du contenant (3) suite au prélèvement du fluide corporel (2) hors du contenant (3) par l'unité de prélèvement (6), du fait de la pression différentielle entre l'espace intérieur (5) du contenant (3) et l'environnement (7), le couvercle (4) et / ou le contenant (3) étant déformable ou déformables de telle sorte, que le kit (1) puisse être transféré de la position d'étanchéité dans la position d'aération, dans la position d'aération du kit (1), de l'air provenant de l'environnement (7) affluant dans l'espace intérieur (5) du contenant (3) et aérant l'espace intérieur (5) du contenant (3),
**caractérisé en ce que**
le couvercle (4) comporte au moins un élément d'aération (8), qui lorsque le kit (1) se trouve dans la position d'aération, suite à la déformation du couvercle (4) et / ou du contenant (3), du fait d'un contact avec le contenant (3) et / ou le couvercle (4), créé dans le siège d'étanchéité, entre le couvercle (4) et le contenant (3) au moins une encoche (9), à travers laquelle de l'air provenant de l'environnement (7) peut affluer dans l'espace intérieur (5) du contenant (3).

2. Kit (1) selon la revendication 1, **caractérisé en ce que** l'élément d'aération (8) est conçu sous la forme d'une came (10).

3. Kit (1) selon la revendication 2, **caractérisé en ce que** la came (10) est placée sur une face inférieure (11) du couvercle (4) dirigée vers le contenant (3), lorsque le kit (1) se trouve dans la position d'étanchéité.

4. Kit (1) selon l'une quelconque des revendications précédentes, **caractérisé en ce que** l'unité de prélèvement (6) est conçue de forme tubulaire, sur une première extrémité (12), l'unité de prélèvement (6) étant conçue en étant ouverte et sur une deuxième extrémité (13), étant munie d'une membrane (14) auto-obturatrice, une fois qu'elle a été percée.

5. Kit (1) selon la revendication 4, **caractérisé en ce que** la membrane (14) comporte une zone de perçage (15) prédéfinie, dans la région de la zone de perçage (15), la membrane (14) étant de préférence fendue.

6. Kit (1) selon l'une quelconque des revendications précédentes, **caractérisé en ce que** le contenant (3) est conçu en forme de coupe.

7. Kit (1) selon l'une quelconque des revendications précédentes, **caractérisé en ce que** le couvercle (4) comporte une section transversale de forme circulaire.

8. Kit (1) selon l'une quelconque des revendications précédentes, **caractérisé en ce que** le couvercle (4) peut être maintenu sur le contenant (3) à l'aide d'un moyen de fixation (16), le moyen de fixation (16) étant de préférence un filetage (17).

9. Kit (1) selon l'une quelconque des revendications précédentes, **caractérisé en ce qu'**un diamètre (18) du couvercle (4) est supérieur à un diamètre (19) du contenant (3) (19), de telle sorte que dans la position d'étanchéité du kit (1), le couvercle (4) entoure le contenant (3).

10. Kit (1) selon l'une quelconque des revendications précédentes, **caractérisé en ce qu'**une section transversale d'ouverture (20) de l'unité de prélèvement (20) (6) se situe dans un plan avec une surface (21) du couvercle, notamment avec une face inférieure (11) du couvercle (4).

11. Kit (1) selon l'une quelconque des revendications précédentes, **caractérisé en ce que** le couvercle (4) comporte au moins un conduit de circulation (22) d'air, à travers lequel lorsque le kit (1) se trouve dans la position d'aération, avant ou après avoir traversé l'encoche (9), de l'air peut affluer à partir de l'environnement (7) dans l'espace intérieur (5) du contenant (3).

12. Kit (1) selon la revendication 11, **caractérisé en ce que** dans la position d'étanchéité du kit (1), le conduit de circulation (22) d'air est placé dans une région de contact (23) du couvercle (4) et du contenant (3), notamment dans une surface frontale (30) périphérique, de forme annulaire du contenant (3).

13. Procédé, destiné à établir une compensation de pression entre un espace intérieur (5) d'un contenant (3) d'un kit (1) selon l'une quelconque des revendications 1 à 12 et un environnement (7), comprenant les étapes de procédé suivantes :
- le kit (1) se trouve dans la position d'étanchéité et dans l'espace intérieur (5) du contenant (3) se trouvent un fluide corporel (2) et de l'air,
- une pression dans l'espace intérieur (5) du contenant (3) se réduit suite à un prélèvement d'un fluide corporel (2) à travers l'unité de prélèvement (6) à partir de l'espace intérieur (5), de sorte que par rapport à un environnement (7) du kit (1), une dépression se produise dans l'espace intérieur (5) du contenant (3) et que le couvercle (4) et / ou le contenant (3) du kit (1) soit ou soient déformé(s),
- du fait de la déformation, le kit (1) est transféré de la position d'étanchéité dans la position d'aération, dans laquelle de l'air provenant de l'environnement (7) afflue dans l'espace intérieur (5) du contenant (3) et aère l'espace intérieur (5) du contenant (3), suite à quoi, le kit (1) est retransféré dans la position d'étanchéité.

14. Procédé selon la revendication 13, **caractérisé en ce que** le kit (1) est transféré automatiquement entre la position d'étanchéité et la position d'aération.
